# EUROPÄISCHE PATENTSCHRIFT

(11) **EP 1 107 988 B1**
(45) Veröffentlichungstag und Bekanntmachung des Hinweises auf die Patenterteilung: **12.12.2007**
(21) Anmeldenummer: 99944373.2
(22) Anmeldetag: 11.08.1999
(51) Int. Cl.: C07K 14/62

(54) **VERFAHREN ZUR CHROMATOGRAPHISCHEN REINIGUNG VON INSULINEN**
METHOD FOR CHROMATOGRAPHICALLY PURIFYING INSULINS
PROCEDE DE PURIFICATION CHROMATOGRAPHIQUE D'INSULINES

(30) Priorität: 24.08.1998 DE 19838097
(43) Veröffentlichungstag der Anmeldung: 20.06.2001
(73) Patentinhaber: Sanofi-Aventis Deutschland GmbH, 65929 Frankfurt am Main (DE)
(72) Erfinder: SIEVERS, Werner, D-65929 Frankfurt (DE); BICKER, Richard, D-68358 Liederbach (DE); DESCH, Dieter, D-60529 Frankfurt (DE); VON EYSMONDT, Jörg, D-65719 Hofheim (DE); KELLER, Reinhold, D-65812 Bad Soden (DE); RICHARD, Frank, D-61476 Kronberg (DE)
(86) Internationale Anmeldenummer: PCT/EP1999/005887
(87) Internationale Veröffentlichungsnummer: WO 2000/011030

(56) Entgegenhaltungen:
- EP-A- 0 474 213
- EP-A- 0 547 544
- GB-A- 1 285 024

## Beschreibung

Die vorliegende Erfindung betrifft ein verbessertes Verfahren zur chromatographischen Reinigung von Insulinen.

Die Herstellprozesse für Insuline sind neben enzymatischen und/oder gentechnischen Verfahren im wesentlichen durch chromatographische Verfahren gekennzeichnet, um die extrem hohen Reinheitsanforderungen zu erfüllen.

Unter dem Begriff Insuline werden hier aus natürlichen Quellen stammende oder rekombinante (d.h. von genetisch modifizierten Mikroorganismen exprimierte) Insuline tierischen oder menschlichen Ursprungs (z.B. Schweineinsulin, Rinderinsulin oder Humaninsulin), Proinsuline (z.B. Insulinvorprodukte, Präinsuline), oder Insulinderivate verstanden.

Als Insulinderivate werden im folgenden Derivate von natürlich vorkommenden Insulinen, nämlich Humaninsulin oder tierischen Insulinen, bezeichnet, welche sich durch Substitution wenigstens eines natürlich auftretenden Aminosäurerestes und/oder Addition wenigstens eines Aminosäurerestes und/oder organischen Restes von dem entsprechenden, ansonst gleichen natürlich vorkommenden Insulin unterscheiden.

Humaninsulin ist ein Polypeptid, das aus 51 Aminosäuren aufgebaut ist. Die sogenannte A (acidic) Kette besteht aus 21, die B (basic) Kette aus 30 Aminosäureresten. In den beiden Aminosäureketten kommen 6 Cysteinreste vor, wobei je zwei Cysteinreste über eine Disulfidbrücke untereinander verbunden sind (die beiden Ketten werden durch zwei Cysteinbrücken miteinander verknüpft). Im biologisch aktiven Humaninsulin sind die A- und B-Ketten über zwei Cysteinbrücken miteinander verbunden, und eine weitere Brücke kommt in der A-Kette vor. Die folgenden Cysteinreste sind im (biologisch wirksamen) Humaninsulin miteinander verknüpft:
A6 - A11
A7 - B7
A20 - B19

Die Buchstaben A oder B stehen für die jeweilige Insulinaminosäurekette, die Zahl für die Position des Aminosäurerestes, die vom Amino- zum Carboxylende der jeweiligen Aminosäurekette gezählt wird.

Die Herstellung von rekombinanten Insulin erfolgt üblicherweise in den Schritten Fermentation und Zellaufschluß, gefolgt von proteinchemischen und verfahrenstechnischen, üblicherweise chromatographischen Prozessen zur Aufreinigung des Produktes.

Gentechnische Verfahren erlauben es, menschliches Proinsulin oder Proinsulin (Proinsulin von Insulinderivaten), das eine von Humaninsulin abweichende Aminosäuresequenz und/oder Aminosäurekettenlänge hat, in Mikroorganismen herzustellen. Die von genetisch veränderten Escherichia-coli-Zellen hergestellten Proinsuline haben keine korrekt verbundenen Cysteinbrücken. Ein Verfahren zur Gewinnung von Humaninsulin mit korrekt verbundenen Cysteinbrücken mit E. coli ist beispielsweise aus der EP 0 055 945 bekannt. Verbesserte Verfahren zur Herstellung von Humaninsulin und Insulinderivaten mit korrekt verbundenen Cysteinbrücken sind in der EP 0 600 372 A1 (US 5,473,049) und in der EP 0 668 292 A2 (US 5,663,291) beschrieben.

Das von genetisch modifizierten Mikroorganismen hergestellte Proinsulin, ein Vorläufer von Insulin, wird zunächst aus den Zellen isoliert, korrekt gefaltet und dann enzymatisch zum Humaninsulin umgewandelt. Das bei den enzymatischen Peptidierungsprozessen anfallende Spaltungsgemisch enthält neben unerwünschten Nebenprodukten sowohl den Wertstoff als auch unerwünschte insulinähnliche Begleitstoffe, die sich weder im Molekulargewicht noch in anderen physikalischen Eigenschaften deutlich vom Wertprodukt unterscheiden, was die Abtrennung und Reinigung gerade im großtechnischen Maßstab sehr erschwert.

Die verfahrenstechnischen Prozesse zur Aufreinigung sind verschiedene, hintereinandergeschaltete Chromatographieverfahren (z.B. Adsorptionschromatographie, lonenaustauschchromatographie, Reversed Phase bzw. Umkehrphasen-Hochdruck-Chromatographie oder Kombinationen daraus) z.T. in mehreren Stufen mit unterschiedlichen Trägermaterialien, z.T. mit anschließender Kristallisation, wobei die eigentliche Reinigung durch Chromatographie erzielt wird. Die Abtrennung der insulinähnlichen Begleitstoffe findet dabei an Ionenaustauschern oder an Reversed Phase Kieselgelträgern statt.

Das End-polishing (Abtrennen geringster Verunreinigungen, als letzte Reinigungsstufe) wird üblicherweise im Hochdruckbereich mit einer Chromatographie an Reversed Phase Kieselgel durchgeführt (RP-HPLC = Reversed Phase High Pressure Liquid Chromatography).

Unter Reversed Phase- (bzw. Umkehrphasen-, d.h. lipophil modifiziertes, also hydrophobes) Kieselgel wird ein Silika-Material verstanden, auf das eine hydrophobe Matrix aufgebracht wurde. Beispiele für eine hydrophobe Matrix sind Alkane mit einer Kettenlänge von 3 bis 20 Kohlenstoffatomen, insbesondere 4 bis 18 Kohlenstoffatomen. Die Korngrößen liegen im Bereich von 10 bis 50 µm, die Porenweiten bei 50 bis 300 A.

Beispiele für Chromatographie-Verfahren, die gemäß dem Stand der Technik RP-Kieselgele (lipophil modifizierte Kieselgele) nutzen, sind EP 0 547 544 A2 (US 5,621,073) oder EP 0 474 213 A1 (US 5,245,008). Nach dem Stand der Technik können nur durch die Verwendung von Reversed Phase Kieselgelen die hohen Anforderungen an die Reinheit der herzustellenden Insuline erfüllt werden. Die Verwendung von Reversed Phase Kieselgel hat aber entscheidende Nachteile:

Reversed Phase Kieselgele sind nur im Bereich von pH 2 bis pH 10 stabil. Bei der Chromatographie von Fermentationsprodukten sind immer hochmolekulare Nebenprodukte enthalten, die nachhaltig adsorbiert werden und mit der üblichen Elution nicht desorbiert werden können. Diese Nebenprodukte reichern sich mit der Zeit auf dem RP-Kieselgel an (man spricht von der Alterung des Adsorbens). Eine Regeneration bzw. ein Cleaning in place (CIP) gelingt meist nur durch Spülung mit verdünnter Natronlauge. Somit wird bei jedem CIP-Vorgang ein Teil des RP-Kieselgeles zerstört, dessen stetiger Ersatz sehr kostenintensiv ist. Ferner besteht für Insuline auf Kieselgelen Denaturierungsgefahr.

Es sind viele Versuche bekannt, RP-Kieselgele auf Silikabasis zu ersetzen. Versuche mit RP-Material auf Aluminiumoxid- oder Titandioxid-Basis (beide Materialien sind nicht voll pH-stabil, aber zumindest stabiler als Silikagel) haben gezeigt, daß die Trennung nur ungenügend ist und daß die geforderten Spezifikationen bzgl. der Reinheit nicht zu erreichen sind.

Eine weitere notwendige Eigenschaft von Chromatographiematerialien ist deren Druckstabilität. Unter druckstabilen polymeren Chromatographiematerialien werden Partikel (die in allen möglichen Formen wie in Stäbchenform, in Form von Bruchstücken oder vorzugsweise in Kugelform vorliegen können und vorzugsweise Durchmesser zwischen 10 und 35 µm aufweisen) von organischen Polymeren verstanden, deren Deformation unter Druckeinwirkung (bis 70 bar) nur gering ist. Das in der Chromatographie-Säule befindliche Material muß so gut gepackt sein, daß keine Hohlräume vorhanden sind (die Qualität der Packung entscheidet über das Trennergebnis). Zum Packen von Kolonnen sind grundsätzlich zwei verschiedene Techniken bekannt, die auch in Kombination angewendet werden können. Es gibt die Methode, mittels eines (meist hydraulisch betätigten) Stempels die Packung zusammen zu drücken (DAC = Direct Axial Compression), bzw. mittels einer Hochdruckpumpe die Kolonne hydrodynamisch zu packen, d.h. eine Suspension aus Flüssigkeit und Partikeln in die Kolonne zu drücken. In beiden Fällen zeigt die Praxis, daß Drücke von bis zu 70 bar auf den Querschnitt der Kolonne wirken müssen, um die Partikel möglichst dicht und ohne entstehende Hohlräume packen zu können.

Viele organische Polymer-Partikel sind nicht druckstabil und deformieren sich unter Druckeinwirkung soweit, daß aus Kugeln flache Scheiben werden, die sich überlappen, und den Durchfluß durch die Packung unterbinden. Demgegenüber sind Reversed Phase-Kieselgele von Natur aus wesentlich härter, und deformieren sich unter den genannten Drücken kaum.

Aufgabe der vorliegenden Erfindung ist es, ein Verfahren zur chromatographischen Reinigung von Insulinen an geeigneten Chromatographiematerialien, die druckstabil sind, bereitzustellen, welches eine so hohe Trennleistung erbringt, daß anstelle der nach dem Stand der Technik üblichen zwei oder mehreren hintereinander geschalteten Chromatographiestufen zur Erzielung der erforderlichen Reinheit des Insulins unter gleichzeitiger Erhöhung der Ausbeute dieses Reinigungsschritts lediglich eine Stufe benötigt wird.

Die Aufgabe wird gelöst durch ein Verfahren zur chromatographischen Reinigung von Insulinen, welches sich dadurch auszeichnet, daß ein druckstabiles organisches polymeres Chromatographiematerial für die Reversed Phase Chromatographie als stationäre Phase verwendet wird, die mobile Phase für die Elution mindestens ein mit Wasser mischbares organisches Lösungsmittel und mindestens eine Puffersubstanz enthält und der pH-Wert 7 bis 11 beträgt, wobei die Partikel des Chromatographiematerials aus organischen Polymeren bestehen, deren Deformation unter Druckeinwirkung bis 70 bar nur gering ist.

Überraschenderweise konnte gefunden werden, daß mit einer Chromatographie im pH-Bereich von 7 bis 11, also im basischen Bereich, eine sehr gute Trennung auf druckstabilen organischen polymeren Chromatographiematerialien erzielt wird. Vorzugsweise beträgt der pH-Wert 9 bis 10.

Ein besonderer Vorteil des Verfahrens gemäß der vorliegenden Erfindung besteht darin, daß in diesem basischen pH-Bereich die Bildung von Des-Amido-Insulin unterdrückt wird, einer Begleitsubstanz, die üblicherweise im sauren Milieu entsteht und gemäß den Spezifikationen von Insulinzubereitungen auf sehr geringe Restmengen abzutrennen ist.

Die mobilen Phasen, die für die Elution eingesetzt werden, enthalten mit Wasser mischbare organische Lösungsmittel, wie beispielsweise Alkohole mit 1 bis 4 Kohlenstoffatomen, Ketone, Methylacetat oder Acetonitril. Bevorzugt sind Alkohole wie 1- oder 2-Propanol (n- oder iso-Propanol), Methanol oder Ethanol. Die Konzentration der mit Wasser mischbaren organischen Lösungsmittel liegt zwischen 1 und 90 Vol.%, bevorzugt zwischen 10 und 50 Vol.%.

Die mobilen Phasen enthalten weiterhin eine Puffersubstanz, um den pH-Wert des Elutionsmittels konstant zu halten. Geeignete Puffersubstanzen sind z.B. Phosphate, Alkali- oder Erdalkalimetallsalze, wie Natriumcitrat oder Kaliumacetat, Ammoniumcitrat, -acetat, - sulfat oder -chlorid.

Die Einstellung des pH-Wertes erfolgt duch die Zugabe von Salzsäure oder Natronlauge.

Die Elution kann isokratisch, d.h. mit konstanter Konzentration der Puffersubstanzen und mit konstantem Anteil des organischen Lösungsmittels, oder bevorzugt mit einem linearen Gradienten, also mit einer Erhöhung des Lösungsmittelanteiles erfolgen.

Die durchschnittliche Partikelgröße des druckstabilen organischen polymeren Chromatographiematerials sollte vorteilhafterweise 5 bis 300 µm, vorzugsweise 10 bis 50 µm betragen. Je kleiner die Partikelgröße, desto schärfer und besser wird die Trennung. Allerdings ist die Druckstabilität von kleineren Partikeln geringer.

Insulin ist ein relativ kleines Polypeptid (Molekulargewicht ca. 6000) und kann problemlos in Poren mit einem Durchmesser von 10 nm diffundieren (keine sterische Hinderung). Materialien mit kleinen Porendurchmessern sind geeigneter, da die spezifische Oberfläche und damit die Adsorptionskapazität größer sind. Die durchschnittliche Porengröße des druckstabilen organischen polymeren Chromatographiematerials beträgt vorteilhafterweise 5 bis 500 nm, vorzugsweise 10 bis 50 nm.

Für das Verfahren gemäß der vorliegenden Erfindung sind druckstabile organische polymere Chromatographiematerialien, die vorzugsweise aus Poly-Styrol-Divinylbenzol oder aus Polymethacrylat bestehen, besonders geeignet. Beispiele für handelsübliche druckstabile organische polymere Chromatographiematerialien, die bei dem Verfahren gemäß der vorliegenden Erfindung vorteilhaft eingesetzt werden können, sind in Tab. 1 zusammengestellt.

**Tab. 1: Handelsübliche Chromatographiematerialien**

| Hersteller | Handelsname | Material | kleinste Partikelgröße [µm] | Porendurchm. [nm] |
|---|---|---|---|---|
| TosoHaas | Amberchrome® | PMA | 35 | 100 |
| Pharmacia | Source® | StDVB | 15 | 100 |
| Perseptive | Poros® | StDVB | 10 | 200 |
| Mitsubishi | CHP20 P® | StDVB | 35 | 100 |
| Biosepra | RPC PolyBio® | StDVB | 10 | 30 |
| Macherey&Nagel | Nucleogel® | StDVB | 20 | 10 |
| Polymer Laboratories | PLRP® | StDVB | 10-15 | 10 |

| | | | | |
|---|---|---|---|---|
| PMA = Polymethacrylat StDVB = Styrol-Divinylbenzol | | | | |

Das erfindungsgemäße Verfahren eignet sich zur analytischen, zur semipräparativen und insbesondere zur präparativen Chromatographie. Unter dem Begriff "präparative Chromatographie" wird das Herstellen von Reinprodukten im technischen Maßstab verstanden.

Um die für Insulinzubereitungen erforderliche Reinheit zu erzielen, ist es notwendig, der Reversed Phase Chromatographie, etwa gemäß EP 0 547 544 A2 (US 5,621,073) oder EP 0 474 213 A1 (US 5,245,008), mindestens eine weitere Reversed Phase- oder eine Kationenaustausch-chromatographie und gegebenenfalls einen Kristallisationsschritt vorzuschalten. Im erfindungsgemäßen Verfahren wird dasselbe Ergebnis mit einer einstufigen Chromatographie auf dem Polymerträger erreicht. Mit dem erfindungsgemäßen Verfahren wird daher die Gesamtausbeute bei der Insulinherstellung wesentlich verbessert, da durch Zusammenlegen mehrerer Prozeßstufen in eine Stufe Ausbeuteverluste entfallen.

Das Verfahren gemäß der vorliegenden Erfindung ist zur chromatographischen Reinigung sämtlicher Insuline gemäß der eingangs eingeführten Definition geeignet, nämlich aus natürlichen Quellen stammende oder rekombinante (d.h. von genetisch modifizierten Mikroorganismen exprimierte) Insuline tierischen oder menschlichen Ursprungs (z.B. Schweineinsulin, Rinderinsulin oder Humaninsulin), Proinsuline (z.B. Insulinvorprodukte, Präinsuline), oder Insulinderivate, wobei unter Insulinderivaten Derivate von natürlich vorkommenden Insulinen, nämlich Humaninsulin oder tierischen Insulinen, verstanden werden, welche sich durch Substitution wenigstens eines natürlich auftretenden Aminosäurerestes und/oder Addition wenigstens eines Aminosäurerestes und/oder organischen Restes von dem entsprechenden, ansonst gleichen natürlich vorkommenden Insulin unterscheiden.

Beispiele für solche Insuline sind Humaninsulin, Rinderinsulin, Schweineinsulin, Insuline gemäß EP 0 368 187 (US 5,656,722), beispielsweise Gly(A21),Arg(B31), Arg(B32)-Humaninsulin, Insuline gemäß EP 0 821 006 (ZA 97/6645) oder die in der EP 0 547 544 A1 (US 5,621,073), EP 0 474 213 A1 (US 5,245,008), EP 0 600 372 A1 (US 5,473,049) oder in der EP 0 668 292 (US 5,663,291) beschriebenen Insuline. (Die Buchstaben A und B stehen für die jeweilige Insulinaminosäurekette, die Zahl für die Position des natürlich auftretenden Aminosäurerestes, welcher durch den vor der Klammer angegebenen Aminosäurerest ausgetauscht ist.)

### Beispiele

### Beispiel 1: Variation des pH-Wertes

In Beispiel 1 wurden Versuche an einer semipräparativen Säule der Abmessungen 10 mm im Durchmesser und 120 mm Länge gemacht, die mit PLRP-S 10-15µm 100A (Polymer Laboratories) gefüllt ist. Die Aufgabe bestand darin, vorgereinigtes Insulin, welches eine Reinheit von 95 Flächen-% hat, derart aufzureinigen, daß die Reinheit größer 98,5 Flächen-% betrug.

Die Aufgabemenge wurde so eingestellt, daß sich eine Beladung des Polymer-Chromatographiematerial von 6 g / Liter [Bettvolumen] ergab. Der Auftragepuffer und die mobile Phase sind Wasser-Propanol-Mischungen, mit 0,05 m Ammoniumacetat und 0,1 m Glycin, die mit Salzsäure bzw. mit NaOH auf den jeweiligen pH-Wert eingestellt wurden. Die Leerrohrgeschwindigkeit betrug 150 cm/h. Es wurden die drei Werte pH 3.5 - pH 6.8 - pH 9 eingestellt. Das Eluat wurde in Fraktionen gesammelt.

In Tabelle 2 sind die Ergebnisse dargestellt. Nur bei pH 9 werden Reinheiten über 98 Flächen-% erreicht, und damit die Spezifikationen erfüllt. Eindeutig ist zu erkennen, daß die Insulinreinigung auf polymeren Chromatographiematerialien nur im basischen Milieu die geforderte Effizienz aufweist.

**Tab. 2: Reinheit und Konzentration von Insulin bei unterschiedlichen pH-Werten mit dem Polymer-Chromatographiematerial PLRP-S 10-15 100**

| | **pH 3.5** | | **pH 6.8** | | **pH 9** | |
|---|---|---|---|---|---|---|
| Fraktion | Reinheit [FI.-%] | Konz. [mg/ml] | Reinheit [FI.-%] | Konz. [mg/ml] | Reinheit [FI.-%] | Konz. [mg/ml] |
| 1 | | | 95,22 | 0,236 | 92,74 | 0,565 |
| 2 | 96,74 | 0,449 | 94,68 | 0,196 | 96,70 | 1,310 |
| 3 | 96,69 | 0,690 | 94,84 | 0,274 | 98,59 | 1,362 |
| 4 | 97,43 | 1,020 | 95,38 | 0,454 | 99,54 | 2,020 |
| 5 | 97,39 | 1,240 | 96,84 | 1,120 | 98,99 | 2,230 |
| 6 | 97,14 | 1,330 | 97,95 | 2,236 | 99,24 | 1,730 |
| 7 | 96,07 | 1,250 | 97,82 | 3,140 | 98, 80 | 1,340 |
| 8 | 94,94 | 1,120 | 97,64 | 3,120 | 98,46 | 1,200 |
| 9 | 93,30 | 0,974 | 97,12 | 2,080 | 97,28 | 0,960 |

### Beispiel 2: Druckstabilität und Packen einer Säule

Es wurde eine Chromatographie-Säule (Prochrom® LC50, mit 50 mm Durchmesser) mit der DAC (Direct Axial Compression) - Technik gepackt. Das Chromatographiematerial (stationäre Phase) wurde in 100% Methanol vorgelegt und in die Säule gepackt. Bei verschiedenen Flußraten wurde der Druckverlust eines 11 %-n-Propanol-Gemisches gemessen. Der Stempeldruck der Chromatographie-Kolonne wurde zwischen 5 und 80 bar variiert.

Folgende Chromatographie-Materialien wurden untersucht:
PLRP-S 10-15 100® (Polymer Laboratories)
Source® 15 RPC (Amersham Pharmacia Biotech)
Kromasil® 13-120 (Akzo Nobel)

Die Materialien haben etwa gleiche Partikeldurchmesser (spherische Partikel). PLRP® und Source® sind Polymere, das Kromasil® ist ein hochwertiges RP-Kieselgel.

**Tab. 3: Druckverlust in [bar/cm] einer Propanol-Wasser-Mischung**

| | Stempeldruck | | Stempeldruck | | Stempeldruck | | Stempeldruck |
|---|---|---|---|---|---|---|---|
| | **5 bar** | | **20 bar** | | **40 bar** | | **80 bar** |
| cm/h | PLRP® | Source® | PLRP® | Source® | PLRP® | Source® | Kromasil® |
| 50 | 0,31 | 0,40 | 0,32 | 0,85 | 0,50 | 3,33 | 0,5 |
| 100 | 0,62 | 0,80 | 0,72 | 1,85 | 1,08 | 6,33 | 1,0 |
| 150 | 1,00 | 1,50 | 1,04 | 3,14 | 1,67 | 9,33 | 1,6 |

Ein spezifischer Druckverlust von 1 bar/cm bedeutet einen Druckabfall von 30 bar in einer 30 cm hohen Packung, was in der technischen Chromatographie üblich ist.

### Beispiel 3: Reinigung von Humaninsulin im präparativen Maßstab

Im folgenden sind insgesamt 3 Beispiele beschrieben, in denen Humaninsulin in einer technischen Säule, die nach dem DAC-Prinzip mit einem verschiebbaren Stempel gepackt wird, gereinigt wird. Verwendet wurde eine Prochrom®-Säule, Typ LC50. Für alle Versuche hat die Packung die jeweils gleiche Dimension von

Durchmesser 50 mm, Bettlänge 110 bis 120 mm.

Humaninsulin mit einer Reinheit von 95 Flächen-% sollte auf eine Reinheit von größer 98,5 FI.-% gebracht werden.

Verwendet wurden drei Chromatographieträger:
PLRP-S 10-15 100® (Polymer Laboratories)
Source® 15 RPC (Amersham Pharmacia Biotech)
Kromasil® C4 13-120 (Akzo Nobel)

Wie schon in Beispiel 2 beschrieben, sind PLRP und Source® Polymermaterialien, während Kromasil® ein hochwertiger RP-Kieselgelträger ist.

Der Auftragepuffer und die mobile Phase entsprechen den Angaben in Beispiel 1. Die Beladung ist angegeben in Gramm Humaninsulin pro Liter Bettvolumen. Unter Ausbeute wird der Anteil des Eluates verstanden, der eine Reinheit von größer 98,5 Flächen-% aufweist.

**Tab. 4: Ausbeute bei der präparativen Reinigung von Humaninsulin**

| Träger | Beladung | pH | Stempeldruck | Ausbeute |
|---|---|---|---|---|
| PLRP-S 10-15 100® | 6 g/L BV | 9 | 40 bar | **60 %** |
| Source® 15 RPC | 6 g/L BV | 9 | 25 bar | **73 %** |
| Kromasil® C4 13-120 | 6 g/L BV | 3,5 | 80 bar | **68 %** |

In Tab. 4 sind die erzielten Ausbeuten gegenübergestellt. Die Werte von 60 bis 70% sind für eine Bettlänge von ca. 12 cm überraschend gut. Der entscheidende Unterschied zwischen dem Stand der Technik (Reinigung mit Kieselgelträger, hier Kromasil®) und der Chromatographie mit einem Polymerträger ist der pH-Unterschied: Nur bei einem pH-Wert von 9 sind Ausbeuten in dieser Höhe zu erreichen.

### Beispiel 4: Reinigung von Bolus-Insulin im präparativen Maßstab

In diesem Beispiel sollte ein Bolus-Insulin (Fast-Acting-Insulin) gereinigt werden. Das Beispiel soll darüberhinaus demonstrieren, daß als Ausgangslagen für diese Chromatographiestufe auch schlechtere Qualitäten zulässig sind. Der Stand der Technik ist, daß die End-Reinigung von Insulin üblicherweise in zwei Chromatographiestufen durchgeführt wird. Wird die End-polishing-Stufe direkt mit einer schlechten, d.h. stark verunreinigten Ware beladen, können die geforderten Reinheiten und gleichzeitig hohe Ausbeuten nicht mehr erreicht werden.

Überraschenderweise konnte nun gefunden werden, daß das untersuchte PolymerMaterial diese Reinheit in einem einzigen Chromatographieschritt erreicht, was auf die kleinen Partikeldurchmesser von 10 bis 15 µm und die ausgezeichneten Adsorptionseigenschaften zurückzuführen ist.

Es wurden vier Versuche gemacht, mit folgenden Ausgangslagen:
75 Flächen-% Reinheit
85 Flächen-% Reinheit
89 Flächen-% Reinheit
93 Flächen-% Reinheit

In Tab. 5 sind die erzielten Ausbeuten (d.h. derjenige Anteil des eingesetzten Insulins, der mit einer Reinheit von größer 98,5 Flächen-% eluiert wurde) zusammengestellt. Hat die Ausgangslage nur 75 Flächen-%, so wird keine Reinheit über 98,5 Fl.-% erreicht. Die Reinheit liegt im Bereich von nur 98,0 Flächen-%.

Liegen die Ausgangslagen jedoch über 85 Flächen-%, so werden zuverlässig die geforderten Reinheiten von größer 98,5% erzielt, bei Ausbeuten zwischen 60 und 80%.

Die präparativen Bedingungen waren wie in Beispiel 1 beschrieben. Alle Versuche wurden in einer Prochrome®-Säule Typ LC50 mit 50 mm Durchmesser und 12 cm Packungshöhe durchgeführt. Die Beladung war jeweils 6 g/L BV, der pH-Wert wurde auf 9 eingestellt, und der Stempeldruck wurde mit 35 bar gemessen.

**Tab. 5: Reinigung von Bolus-Insulinen verschiedener Qualität**

| Träger | Reinheit der Ausgangslage in Flächen-% | Ausbeute (Reinheit größer 98,5 Flächen-%) |
|---|---|---|
| PLRP-S 10-15 100® | 75 FI.-% | 0 % |
| PLRP-S 10-15 100® | 85 FI.-% | 64 % |
| PLRP-S 10-15 100® | 89 FI.-% | 73 % |
| PLRP-S 10-15 100® | 93 FI.-% | 73 % |

Anhand der Tab. 5 ist gut zu sehen, wie abhängig von der Qualität der Ausgangslage die Ausbeute in der Chromatographie zunimmt. Die Versuche zeigen eindeutig, daß die Reinigung in einer einzigen Chromatographiestufe zuverlässig möglich ist.

## Patentansprüche

1. Verfahren zur chromatographischen Reinigung von Insulinen, **dadurch gekennzeichnet, daß** ein druckstabiles organisches polymeres Chromatographiematerial für die Reversed Phase Chromatographie als stationäre Phase verwendet wird, die mobile Phase für die Elution mindestens ein mit Wasser mischbares organisches Lösungsmittel und mindestens eine Puffersubstanz enthält und der pH-Wert 7 bis 11 beträgt, wobei die Partikel des Chromatographiematerials aus organischen Polymeren bestehen, deren Deformation unter Druckeinwirkung bis 70 bar nur gering ist.

2. Verfahren nach Anspruch 1, **dadurch gekennzeichnet, daß** der pH-Wert 9 bis 10 beträgt.

3. Verfahren nach Anspruch 1 oder 2, **dadurch gekennzeichnet, daß** das mit Wasser mischbare organische Lösungsmittel ein Alkohol mit 1 bis 4 Kohlenstoffatomen ist.

4. Verfahren nach Anspruch 3, **dadurch gekennzeichnet, daß** der Alkohol 1-Propanol oder 2-Propanol ist.

5. Verfahren nach Anspruch 3, **dadurch gekennzeichnet, daß** der Alkohol Ethanol ist.

6. Verfahren nach Anspruch 3, **dadurch gekennzeichnet, daß** der Alkohol Methanol ist.

7. Verfahren nach Anspruch 2, **dadurch gekennzeichnet, daß** das mit Wasser mischbare organische Lösungsmittel ein Keton ist.

8. Verfahren nach Anspruch 2, **dadurch gekennzeichnet, daß** das mit Wasser mischbare organische Lösungsmittel Methylacetat ist.

9. Verfahren nach Anspruch 2, **dadurch gekennzeichnet, daß** das mit Wasser mischbare organische Lösungsmittel Acetonitril ist.

10. Verfahren nach einem oder mehreren der Ansprüche 1 bis 9, **dadurch gekennzeichnet, daß** die Konzentration des mit Wasser mischbaren organischen Lösungsmittels in der mobilen Phase 1 bis 90 Vol.% beträgt.

11. Verfahren nach Anspruch 10, **dadurch gekennzeichnet, daß** die Konzentration des mit Wasser mischbaren organischen Lösungsmittels in der mobilen Phase 10 bis 50 Vol.% beträgt.

12. Verfahren nach einem oder mehreren der Ansprüche 1 bis 11, **dadurch gekennzeichnet, daß** die Elution isokratisch erfolgt.

13. Verfahren nach einem oder mehreren der Ansprüche 1 bis 11, **dadurch gekennzeichnet, daß** die Elution mit einem linear erhöhten Gradienten des Anteils an dem mit Wasser mischbaren organischen Lösungsmittel erfolgt.

14. Verfahren nach einem oder mehreren der Ansprüche 1 bis 13, **dadurch gekennzeichnet, daß** das druckstabile organische polymere Chromatographiematerial eine durchschnittliche Partikelgröße von 5 bis 300 µm aufweist.

15. Verfahren nach Anspruch 14, **dadurch gekennzeichnet, daß** die durchschnittliche Partikelgröße von 10 bis 50 µm aufweist.

16. Verfahren nach einem oder mehreren der Ansprüche 1 bis 15, **dadurch gekennzeichnet, daß** die durchschnittliche Porengröße des druckstabilen organischen polymeren Chromatographiematerials 5 bis 500 nm beträgt.

17. Verfahren nach Anspruch 16, **dadurch gekennzeichnet, daß** die durchschnittliche Porengröße 10 bis 50 nm beträgt.

18. Verfahren nach einem oder mehreren der Ansprüche 1 bis 17, **dadurch gekennzeichnet, daß** das druckstabile organische polymere Chromatographiematerial aus einem Polymethacrylat besteht.

19. Verfahren nach einem oder mehreren der Ansprüche 1 bis 17, **dadurch gekennzeichnet, daß** das druckstabile organische polymere Chromatographiematerial aus einem Poly-Styrol-Divinylbenzol besteht.

## Claims

1. A procedure for the chromatographic purification of insulins, wherein a pressure-stable organic polymeric chromatography material for reversed phase chromatography is used as a stationary phase, the mobile phase for elution contains at least one water-miscible organic solvent and at least one buffer substance and the pH is 7 to 11, the particles of the chromatography material consisting of organic polymers whose deformation under the action of pressure up to 70 bar is only slight.

2. The procedure as claimed in claim 1, wherein the pH is 9 to 10.

3. The procedure as claimed in claim 1 or 2, wherein the water-miscible organic solvent is an alcohol having 1 to 4 carbon atoms.

4. The procedure as claimed in claim 3, wherein the alcohol is 1-propanol or 2-propanol.

5. The procedure as claimed in claim 3, wherein the alcohol is ethanol.

6. The procedure as claimed in claim 3, wherein the alcohol is methanol.

7. The procedure as claimed in claim 2, wherein the water-miscible organic solvent is a ketone.

8. The procedure as claimed in claim 2, wherein the water-miscible organic solvent is methyl acetate.

9. The procedure as claimed in claim 2, wherein the water-miscible organic solvent is acetonitrile.

10. The procedure as claimed in one or more of claims 1 to 9, wherein the concentration of the water-miscible organic solvent in the mobile phase is 1 to 90% by volume.

11. The procedure as claimed in claim 10, wherein the concentration of the water-miscible organic solvent in the mobile phase is 10 to 50% by volume.

12. The procedure as claimed in one or more of claims 1 to 11, wherein the elution is carried out isocratically.

13. The procedure as claimed in one or more of claims 1 to 11, wherein the elution is carried out using a linearly increased gradient of the proportion of the water-miscible organic solvent.

14. The procedure as claimed in one or more of claims 1 to 13, wherein the pressure-stable organic polymeric chromatography material has an average particle size of 5 to 300 *µ*m.

15. The procedure as claimed in claim 14, wherein the average particle size is from 10 to 50 µm.

16. The procedure as claimed in one or more of claims 1 to 15, wherein the average pore size of the pressure-stable organic polymeric chromatography material is 5 to 500 nm.

17. The procedure as claimed in claim 16, wherein the average pore size is 10 to 50 nm.

18. The procedure as claimed in one or more of claims 1 to 17, wherein the pressure-stable organic polymeric chromatography material consists of a polymethacrylate.

19. The procedure as claimed in one or more of claims 1 to 17, wherein the pressure-stable organic polymeric chromatography material consists of a polystyrene/divinylbenzene.

## Revendications

1. Procédé de purification chromatographique d'insulines, **caractérisé en ce qu'**on utilise un matériau de chromatographie polymère organique stable à la pression pour la chromatographie en phase inversée comme phase stationnaire, la phase mobile pour l'élution contient au moins un solvant organique miscible à l'eau et au moins une substance tampon et le pH est de 7 à 11, les particules du matériau de chromatographie étant constituées de polymères organiques dont la déformation sous l'effet de la pression à 70 bars n'est que faible.

2. Procédé selon la revendication 1, **caractérisé en ce que** le pH est de 9 à 10.

3. Procédé selon la revendication 1 ou 2, **caractérisé en ce que** le solvant organique miscible à l'eau est un alcool comprenant 1 à 4 atomes de carbone.

4. Procédé selon la revendication 3, **caractérisé en ce que** l'alcool est le 1-propanol ou le 2-propanol.

5. Procédé selon la revendication 3, **caractérisé en ce que** l'alcool est l'éthanol.

6. Procédé selon la revendication 3, **caractérisé en ce que** l'alcool est le méthanol.

7. Procédé selon la revendication 2, **caractérisé en ce que** le solvant organique miscible à l'eau est une cétone.

8. Procédé selon la revendication 2, **caractérisé en ce que** le solvant organique miscible à l'eau est l'acétate de méthyle.

9. Procédé selon la revendication 2, **caractérisé en ce que** le solvant organique miscible à l'eau est l'acétonitrile.

10. Procédé selon l'une ou plusieurs des revendications 1 à 9, **caractérisé en ce que** la concentration en solvant organique miscible à l'eau dans la phase mobile est de 1 à 90% en volume.

11. Procédé selon la revendication 10, **caractérisé en ce que** la concentration en solvant organique miscible à l'eau dans la phase mobile est de 10 à 50% en volume.

12. Procédé selon l'une ou plusieurs des revendications 1 à 11, **caractérisé en ce que** l'élution se déroule de manière isocratique.

13. Procédé selon l'une ou plusieurs des revendications 1 à 11, **caractérisé en ce que** l'élution se déroule avec un gradient augmentant linéairement de la proportion en solvant organique miscible à l'eau.

14. Procédé selon l'une ou plusieurs des revendications 1 à 13, **caractérisé en ce que** le matériau de chromatographie polymère organique stable à la pression présente une grosseur moyenne des particules de 5 à 300 *µ*m.

15. Procédé selon la revendication 14, **caractérisé en ce que** la grosseur moyenne des particules est de 10 à 50 µm.

16. Procédé selon l'une ou plusieurs des revendications 1 à 15, **caractérisé en ce que** la taille moyenne des pores du matériau de chromatographie polymère organique stable à la pression est de 5 à 500 nm.

17. Procédé selon la revendication 16, **caractérisé en ce que** la taille moyenne des pores est de 10 à 50 nm.

18. Procédé selon l'une ou plusieurs des revendications 1 à 17, **caractérisé en ce que** le matériau de chromatographie polymère organique stable à la pression est constitué d'un polyméthacrylate.

19. Procédé selon l'une ou plusieurs des revendications 1 à 17, **caractérisé en ce que** le matériau de chromatographie polymère organique stable à la pression est constitué d'un polystyrène-divinylbenzène.
